# EUROPÄISCHE PATENTSCHRIFT

(11) **EP 1 713 924 B1**
(45) Veröffentlichungstag und Bekanntmachung des Hinweises auf die Patenterteilung: **09.03.2016**
(21) Anmeldenummer: 04804026.5
(22) Anmeldetag: 17.12.2004
(51) Int. Cl.: C12P 13/12, C07C 319/28

(54) **VERFAHREN ZUR ISOLIERUNG VON METHIONIN AUS EINEM FERMENTATIONSÜBERSTAND**
METHOD FOR ISOLATING METHIONINE FROM A FERMENTATION SUPERNATANT
PROCÉDÉ D'ISOLATION DE MÉTHIONINE D'UN SURNAGEANT DE FERMENTATION

(30) Priorität: 18.12.2003 DE 10359668
(43) Veröffentlichungstag der Anmeldung: 25.10.2006
(73) Patentinhaber: Evonik Degussa GmbH, 45128 Essen (DE)
(72) Erfinder: BOY, Matthias, 63225 Langen (DE); KLEIN, Daniela, 68161 Mannheim (DE); SCHRÖDER, Hartwig, 69226 Nussloch (DE)
(86) Internationale Anmeldenummer: PCT/EP2004/014423
(87) Internationale Veröffentlichungsnummer: WO 2005/059155

(56) Entgegenhaltungen:
- WO-A-03/087386
- WO-A-2005/007862
- FR-A- 1 331 847
- US-A- 3 139 386
- "IONIZATION CONSTANTS AND PH VALUES AT THE ISOELECTRIC POINTS OF THE AMINO ACIDS IN WATER AT 25 DEG C" CRC HANDBOOK OF CHEMISTRY AND PHYSICS, 1970, Seiten C-741-C-743, XP008041278
- HERMANN, T.: "Industrial production of amino acids by coryneform bacteria" JOURNAL OF BIOTECHNOLOGY, Bd. 104, Nr. 1-3, 4. September 2003 (2003-09-04), Seiten 155-172, XP001184757

## Beschreibung

Die vorliegende Erfindung betrifft ein Verfahren zur fermentativen Herstellung von Methionin, ein Verfahren zur Isolierung des dabei gebildeten Methionins, die bei der Isolierung anfallende methioninhaltige Biomasse, deren Verwendung zur Herstellung eines Futter- oder Futterergänzungsmittels, sowie die Verwendung des isolierten Methionins zur Herstellung von Nahrungs- oder Futtermitteln oder Nahrungs- oder Futterergänzungsmitteln.

### Stand der Technik

Methionin findet Verwendung auf verschiedensten Gebieten, einschließlich der Nahrungsmittel-, Futtermittel-, Kosmetik- und pharmazeutischen Industrie.

Bisher sind nur die chemischen Herstellungsverfahren für D,L-Methionin von technischer Bedeutung. Einsatzstoffe für diese Synthese sind Schwefelwasserstoff, Methylmercaptan, Acrolein, Blausäure oder Methylmercaptopropionaldehyd (vgl. Ullmann's Encyclopedia of Industrial Chemistry (1985), Vol. A2, Seite 71).

Methionin wird auch über natürliche zelluläre Stoffwechselprozesse produziert. Seine Produktion in industriellem Maßstab würde am zweckmäßigsten mittels Bakterienkulturen erfolgen, die entwickelt wurden, um große Mengen der gewünschten Substanz zu produzieren und sekretieren. Für diesen Zweck besonders geeignete Organismen sind nicht-pathogene coryneforme Bakterien.

Es ist bekannt, dass Methionin durch Fermentation von Stämmen coryneformer Bakterien, insbesondere Corynebacterium glutamicum, hergestellt werden kann. Wegen der großen Bedeutung wird ständig an der Verbesserung der Herstellverfahren gearbeitet. Verfahrensverbesserungen können z.B. fermentationstechnische Maßnahmen, die Zusammensetzung der Nährmedien, oder die intrinsischen Leistungseigenschaften des Mikroorganismus selbst betreffen.

Zur Verbesserung der Leistungseigenschaften dieser Mikroorganismen hinsichtlich der Produktion eines bestimmten Moleküls können Methoden der Mutagenese, Selektion und Mutantenauswahl oder Methoden der rekombinanten DNA-Technik zur Stammverbesserung von Aminosäure produzierenden Stämmen, wie z.B. von Corynebacterium, eingesetzt werden, indem man einzelne Aminosäure-Biosynthesegene amplifiziert oder ausschaltet und damit eine Verbesserung der Aminosäure-Produktion herbeiführt.

So beschreibt z.B. die WO-A-02/10209 und die DE-A-101 36 986 ein Verfahren zur fermentativen Herstellung von L-Methionin unter Verwendung L-Methionin produzierender genetisch veränderter coryneformer Bakterien. Darin wird unter anderem ein Verfahren zur Herstellung von L-Methionin, umfassend die Fermentation der Bakterien, die Anreicherung der Aminosäure im Medium oder in den Bakterien und die Isolierung der Aminosäure beschrieben. Außerdem ist ein Verfahren zur Herstellung von L-Methionin-haltigem Tierfuttermitteladditiv aus einer Fermentationsbrühe beschrieben, welches folgende Schritte umfasst: a) Fermentation von L-Methionin produzierenden Mikroorganismen; b) Aufkonzentrieren der Fermentationsbrühe; z.B. durch Eindampfen; c) Abtrennung der Biomasse (0 - 100 %), z.B. durch Zentrifugation; und d) die Trocknung, z.B. durch Gefrier- oder Sprühtrocknung, Sprühgranulation.

US3,139,386 beschreibt ein Verfahren zur Isolierung von fermentativ hergestelltem L- Methionin, wobei eine methioninhaltige Fermentationsbrühe auf 100°C erhitzt wird und L-Methionin aus der Brühe abgetrennt und auskristallisiert wird.

Swapan et al beschreiben in J. Microbial Biotechnology, 4 (1), 35-41 (1989) die mikrobielle Produktion von Methionin mittels einer Bacillus megaterium-Mutante, durch Abtrennung der Zellen aus der Fermentationsbrühe, pH-Einstellung auf 5, Behandlung mit Aktivkohle und Ionenaustauscher-Chromatographie.

Aus der DE-A-35 33 198 ist die fermentative Herstellung von L-Leucin mittels spezieller thermophiler Bakterien bekannt. Die Fermentation erfolgt bei +60°C kontinuierlich unter Biomasserückhaltung, Abscheidung von Produkt-haltigem, erschöpftem Medium, Abkühlung (bis auf +2°C) in einem Kristallisator, Gewinnung der auskristallisierten Aminosäure und Rückführung der Mutterlauge in den Reaktor. Die fermentative Methionin-Produktion wird darin nicht beschrieben.

Die bisher beschriebenen Verfahren zur mikrobiellen Produktion von Methionin genügen noch nicht den Anforderungen einer Produktion im technischen Maßstab. Grund dafür ist einerseits die begrenzte Löslichkeit vom Methionin im wässrigen Fermentationsmedium, die bewirkt, dass bei hoher Biosyntheseleistung Methionin in der Fermentationsbrühe ausfällt und somit die Reinigung erschwert. Weiterer Grund ist, dass bei Arbeiten gemäß Stand der Technik beträchtliche Abfallströme anfallen, deren Beseitigung mit hohen Kosten verbunden ist.

### Kurze Beschreibung der Erfindung

Aufgabe der vorliegenden Erfindung war es daher, ein verbessertes Verfahren zur Isolierung von fermentativ hergestelltem Methionin bereitzustellen, welches insbesondere auf solche Fermentationsbrühen anwendbar ist, die Methionin zum Teil in kristalliner Form enthalten. Eine weitere Aufgabe bestand darin, ein Aufarbeitungsverfahren für methioninhaltige Fermentationsbrühen bereitzustellen, das praktisch keine Abfallströme erzeugt und somit besonders wirtschaftlich durchgeführt werden kann.

Obige Aufgabe wurde überraschend durch Bereitstellung eines Aufarbeitungsverfahrens gelöst, das gezielt die Löslichkeitseigenschaften des Methionin zur Abtrennung der Biomasse ausnutzt. Das Verfahren nutzt die Kristallisation als Reinigungsmethode für fermentativ hergestelltes L-Methionin. Es liefert zwei unterschiedliche Produkte für die Anwendung als Feedadditiv (niedrig und hochkonzentriertes Produkt). In bevorzugten Varianten fallen praktisch keine Abfallströme an und erlauben so eine besonders wirtschaftliche Methioninproduktion in technischem Maßstab.

### Detaillierte Beschreibung der Erfindung

### A) Allgemeine Definitionen

"Methionin" im Sinne der Erfindung umfasst grundsätzlich, L- oder D-Methionin, Mischungen dieser Isomeren, wie z.B. Racemate, vorzugsweise aber L-Methionin.

Die Löslichkeit von Methionin beträgt bei 20°C in Wasser etwa 30 g/l, bei 70°C liegt sie über 90 g/l. In der Fermentationsbrühe werden unter diesen Bedingungen Löslichkeiten von vergleichbarer Größenordnung beobachtet.

Verfahrensmaßnahmen, wie "Aufkonzentrieren", "Abtrennen", "Waschen" "Trocknen" umfassen im Sinne der vorliegenden Erfindung sämtliche im Bereich des fachmännischen Könnens liegende Verfahren. So kann beispielsweise unter "Aufkonzentrieren" ein Eindampfen der Flüssigphase unter Normaldruck oder Anlegen eines Vakuums verstanden werden. Ein "Aufkonzentrieren" kann beispielsweise unter Anwendung gängiger Techniken, wie der Umkehrosmose oder der Nanofiltration oder gängiger Vorrichtungen, wie z.B. eines Fallfilmverdampfers, Dünnschichtverdampfers oder Rotationsverdampfers oder Kombinationen davon erfolgen. Ein "Abtrennen" kann beispielsweise eine Zentrifugation, eine Filtration, ein Dekantieren oder Kombinationen dieser Verfahren umfassen. Ein "Waschen" kann beispielsweise das Abfiltrieren eines Feststoffs und ein- oder mehrmaliges Waschen, gegebenenfalls nach Aufschlämmen des Filterrückstandes, umfassen. Ein "Trocknen" kann beispielsweise eine Gefriertrocknung, Sprühtrocknung, Sprühgranulation, Wirbelschichttrocknung oder Kombinationen dieser Verfahren umfassen.

### B) Bevorzugte Ausführungsformen der Erfindung

Ein erster Gegenstand der Erfindung betrifft ein Verfahren zur Isolierung von fermentativ hergestelltem Methionin, wobei man a) eine Methionin-haltige, bei der Fermentation eines Methionin-produzierenden Mikroorganismus anfallende flüssige Fraktion, welche insbesondere Methionin in teilweise ungelöster Form enthält, auf eine Temperatur im Bereich von 60 bis 100°C erwärmt, die ausreicht, um die Löslichkeit von Methionin in der Flüssigphase zu erhöhen, vorzugsweise um Methionin im Wesentlichen vollständig in Lösung zu bringen, b) daraus eine an Methionin angereicherte Flüssigphase gewinnt, indem man aus der angereicherten Fermentationsbrühe die Biomasse abtrennt, und c) Methionin, gegebenenfalls nach Aufkonzentrieren der angereicherten Flüssigphase, auskristallisiert,
wobei mann die in Stufe b) abgetrennte Biomasse g1) gegebenenfalls wäscht, wobei die zum Waschen verwendete Flüssigkeit gegebenenfalls erwärmt ist, und g3) trocknet.

"Im Wesentlichen" vollständig in Lösung befindet sich Methionin, wenn es z.B. zu mehr als 95%, mehr als 98% insbesondere zu 100%, bezogen auf das den Gesamtmethioningehalt in der flüssigen Phase, gelöst ist.

Eine Methionin-haltige "flüssige Fraktion" ist typischerweise die aus dem Fermentationsprozess gewonnene Brühe, welche insbesondere Methionin in teilweise ungelöster Form enthält, und gegebenenfalls weitere feste Bestandteile aufweisen kann, die üblicherweise in Fermentationsbrühen enthalten sein können; oder eine davon abgeleitete, z.B. durch geeignete Vorbehandlung erhaltene, Flüssigkeit. Eine "Vorbehandlung" könnte beispielsweise in einer Aufkonzentrierung durch Eindampfen oder in einer Zugabe von Stoffen bestehen. Beispielsweise könnten der Brühe Methionin-haltige Fraktionen aus früheren Aufarbeitungsansätzen zugegeben werden, oder Zusätzen (vgl. unten), welche die folgenden Verarbeitungsschritte oder die bestimmungsgemäße Verwendung des Produkt (z.B. als Feedadditiv) fördern .

Der Gehalt an ungelöstem Methionin in der gegebenenfalls aufgestockten Fermentationsbrühe liegt, bezogen auf das Gesamtgewicht der Fermentationsbrühe im Bereich von etwa 1 bis 10 Gew.-%, vorzugsweise von etwa 3 bis 8 Gew.-% oder bezogen auf den Gesamtfeststoffgehalt im Bereich von etwa 30 bis 80 Gew.-%, vorzugsweise von etwa 50 bis 57Gew.-%.

Beispielsweise kann eine erfindungsgemäße Fermentation einen Methioningehalt von etwa 96 g/l ergeben, wovon bei einer typischen Fermentationstemperatur etwa 46 g/l in Lösung und etwa 50 g/l ungelöst vorliegen.

Der Methioningehalt der angereicherten Flüssigphase liegt, bezogen auf deren Trockenrückstände, im Bereich von etwa 60 bis 100 Gew.-% oder etwa 90 bis 100 Gew.-%, beispielsweise von etwa 75 bis 85 Gew.-% oder etwa 95 bis 100 Gew.-%

Um Methionin im Wesentlichen in Lösung zu bringen erwärmt man in Stufe a) auf eine Temperatur im Bereich von etwa 60 bis 120 °C, vorzugsweise etwa 70 bis 100 ° C, je nach Menge des zu lösenden Produkts. Gegebenenfalls kann es dabei erforderlich sein, unter leicht erhöhtem Druck, wie z.B. 1 bis 5 atm zu arbeiten.

Vorzugsweise setzt man in Stufe a) als flüssige Fraktion die Biomasse-haltige Fermentationsbrühe ohne weitere Vorbehandlung ein.

Die an Methionin angereicherte Flüssigphase der Stufe b) erhält man dadurch, dass man aus der mit gelöstem Methionin angereicherten, erwärmten Fermentationsbrühe die Biomasse abtrennt. Um ein vorzeitiges Auskristallisieren des Methionins zu verhindern, arbeitet man während Biomasseabtrennung ebenfalls bei erhöhter Temperatur, vorzugsweise bei einer Temperatur im oben angegebenen Bereich.

In einer bevorzugten Ausführungsform der Erfindung wird
d) das auskristallisierte Methionin abgetrennt,
e) das abgetrennte feste, vorzugsweise kristallins Methionin gegebenenfalls gewaschen und
f) gegebenenfalls getrocknet.

Ein Erwärmen der Waschflüssigkeit kann dann erforderlich werden, falls z.B. festes Methionin in der abgetrennten Biomasse-Fraktion enthalten sein sollte, und es gewünscht ist, aus der Biomasse-Fraktion Methionin so weit wie möglich zu gewinnen.

Um Abfallströme zu vermeiden, wird vorzugsweise
g2) die in Stufe g1) anfallende Waschflüssigkeit mit der an Methionin angereicherten Flüssigphase von Stufe b) vereinigt.

Die nach obigen Verfahrensweisen erhaltenen Methionin-haitigen Flüssigphasen der Stufe b) werden dann weiter aufkonzentriert, z.8. durch Eindampfen unter Erwärmung und gegebenenfalls Anlegen eines Vakuums. Der Methionin-Gehalt im dabei anfallenden Konzentrat liegt dabei im Bereich von *etwa* 10 bis 40 Gew.-%, bezogen auf das Gesamtgewicht des Konzentrats. Die Abtrennung des Methionins erfolgt dabei vorzugsweise durch Kühlungskristallisation. Dazu wird die Lösung auf Temperaturen im Bereich von 0 bis 20°C abgekühlt. Nach beendeter Kristallisation wird das feste Methionin mit kalter Waschflüssigkeit, z.B. Wasser, gewaschen und, gegebenenfalls unter leichtem Erwärmen, getrocknet.

Nach einer weiteren Verfahrensvariante wird die in Stufe d) anfallende Mutterlauge
d1) mit der Methionin-haitigen flüssigen Fraktion aus einem anderen Fermentationsansatz mit einem Methionin-produzierenden Mikroorganismus vereinigt; oder
d2) der abgetrennten Biomasse aus dem gleichen oder einem anderen Fermentationsansatz mit einem Methionin-produzierenden Mikroorganismus vor dem Trocknen gemäß Stufe g3) zusetzt.

Nach einer weiteren Verfahrensvariante wird die in Stufe e) anfallende Waschflüssigkeit
e1) mit der Methionin-haitigen flüssigen Fraktion aus einem anderen Fermentationsansatz mit einem Methionin-produzierenden Mikroorganismus vereinigt; oder
e2) der abgetrennten Biomasse aus dem gleichen oder einem anderen Fermentationsansatz mit einem Methionin-produzierenden Mikroorganismus vor dem Trocknen gemäß Stufe g3) zusetzt.

Durch Rückführung von Mutterlauge und Waschflüssigkeit wird ein Anfallen von Abfallströmen weiter unterbunden.

Erfindungsgemäß ist es außerdem bevorzugt, dass die Trocknung gemäß Stufe g3) einen Sprühtrocknungsschritt umfasst.

Ein weiterer Gegenstand der Erfindung betrifft ein Verfahren zur fermentativen Herstellung von Methionin, wobei man einen natürlichen oder rekombinanten Mikroorganismus fermentiert und das gebildete Methionin nach einem
Verfahren gemäß obiger Definition isoliert.

In einer bevorzugten Ausführungsform werden die erfindungsgemäßen Verfahren unter Verwendung eines Methionin-produzierenden Mikroorganismus, ausgewählt unter natürliehen oder rekombinanten Bakterien der Gattung Corynebacterium, durchgeführt.

Weiterhin kann das nach obiger Stufe g3) erhältliche Trockengut zur Herstellung eines Futtermittels oder eines Futterergänzungsmittels (Feedadditiv) eingesetzt werden.

Ferner kann das erfindungsgemäß isolierte Methionin zur Herstellung eines Nahrungs- oder Futtermittels oder Nahrungs- oder Futterergänzungsmittels eingesetzt werden.

Die Methionin-haltigen getrockneten Biomassen, erhältlich nach einem Verfahren gemäß obiger Definition, können zur Herstellung von Feedadditiven, umfassend derartige Biomassen sowie von Futtermittelzusammensetzungen, enthaltend neben üblichen Futter- mittelbestandteilen ein solches Feedadditiv eingesetzt werden.

In den folgenden Abschnitten werden weitere Ausgestaltungen der Erfindung beschrieben.

### C) Erfindungsgemäß verwendete Wirtszellen

Für das erfindungsgemäße Verfahren verwendet man vorzugsweise coryneforme Bakterien. Vorzugsweise sind dies Bakterien der Gattung Corynebacterium. Aus der Gattung Corynebacterium ist insbesondere die Art Corynebacterium glutamicum zu nennen, die in der Fachwelt für ihre Fähigkeit bekannt ist, L-Aminosäuren zu produzieren.

Als Beispiele für geeignete Stämme sind zu nennen:
aus der Gattung Corynebacterium:
   Corynebacterium glutamicum ATCC 13032, Corynebacterium acetoglutamicum ATCC 15806, Corynebacterium acetoacidophilum ATCC 13870, Corynebacterium thermoaminogenes FERM BP-1539, Corynebacterium melassecola ATCC 17965;Corynebacterium glutamicum KFCC10065; oder Corynebacterium glutamicum ATCC21608
oder aus der Gattung Brevibacterium:
   Brevibacterium flavum ATCC 14067; Brevibacterium lactofermentum ATCC 13869 und Brevibacterium divaricatum ATCC 14020 zu nennen;
   (KFCC = Korean Federation of Culture Collection; ATCC = American Type Culture Collection; FERM BP = Sammlung des National institute of Bioscience and Human-Technology, Agency of Industrial Science and Technology, Japan)
   Die Bakterienstämme können dabei unverändert oder in geeigneter Weise genetisch verändert eingesetzt werden. So können beispielsweise Mikroorganismen eingesetzt werden, in denen Gene des Methionin-Biosynthesewegs verstärkt werden, so dass mehr Methionin in der Zelle vorliegt. Alternativ oder zusätzlich können auch Gene ausgeschaltet oder abgeschwächt sein, welche in Methionin-abbauende Stoffwechselwege involviert sind. Geeignete Strategien zur Verbesserung der Methionin-Produktion sind aus dem Stand der Technik bekannt und beispielsweise beschrieben in der WO-A-02/10209, DE-A-102 170 58, DE-A-102 393 08, DE-A-102 390 73, DE-A-102 390 82 und DE-A-102 228 58, worauf hiermit ausdrücklich Bezug genommen wird.

Um die Aktivität oder Menge eines Enzyms zu verringern, das den Methioningehalt senken könnte, kann der Fachmann unterschiedliche Maßnahmen einzeln oder in Kombinationen ausführen. Durch Reduktion der Transkriptionshäufigkeit des Gens, das für das erfindungsgemäße Protein kodiert, kann die Konzentration des betreffenden Proteins gesenkt werden. Dies kann der Fachmann durch Veränderung oder Austausch der Promotor- oder Regulationsregion, sowie der Ribosomenbindungsstelle des kodierenden Gens erreichen. Stromab der kodierenden Region kann der Fachmann Terminatoren verändern oder Sequenzen einfügen, die zu einer verringerten Stabilität des Transkriptes führen. Diese, die Lebensdauer der mRNA verringernden Maßnahmen ermöglichen, die Expression des zugehörigen Proteins, und damit seine Konzentration abzusenken.

Auf der Ebene des exprimierten Enzyms können fusionierte Sequenzen zu einer erhöhten Abbaurate und damit ebenfalls zu einer Absenkung der Konzentration des Proteins führen. Außerdem kann der Fachmann durch gezielte oder ungerichtete Mutagenese des kodierenden Gens die Aktivität, die Substrataffinität und die Substratspezifität verändern. Enzyme können durch Mutationen in den korrespondierenden Genen derart in ihrer Aktivität beeinflusst werden, dass es zu einer teilweisen oder vollständigen Verringerung der Reaktionsgeschwindigkeit der enzymatischen Reaktion kommt. Beispiele für solche Mutationen sind dem Fachmann bekannt (Motoyama H. Yano H. Terasaki Y. Anazawa H. Applied & Environmental Microbiology. 67:3064-70, 2001, Eikmanns BJ. Eggeling L. Sahm H. Antonie van Leeuwenhoek. 64:145-63, 1993-94) Mutanten des Proteins können auch zu verringerter oder verhinderter Homo- oder Heteromultimerisierung von Enzymkomplexen und damit ebenfalls zu einer Verschlechterung der enzymatischen Eigenschaften führen.

Solchermaßen veränderte Gene können entweder in Plasmiden oder bevorzugt im Chromosom integriert vorliegen. Dabei kann das ursprüngliche, nicht auf diese Art veränderte Gen noch zusätzlich vorhanden sein, bevorzugt aber gegen das veränderte Gen ausgetauscht sein.

Um die Aktivität eines Enzyms, gemessen in einem coryneformen Bakterium, zu verringern, kann es ausreichend sein, Gene, die für funktionale Äquivalente, wie künstlich hergestellte Mutanten oder natürliche Homologe aus anderen Organismen kodieren, zu exprimieren. Dabei kann das ursprüngliche Gen noch zusätzlich vorhanden sein, bevorzugt aber gegen das veränderte oder homologe Gen ausgetauscht sein.

Zusätzlich kann es für die bakterielle Produktion von Methionin vorteilhaft sein eines oder mehrere Enzyme des Methionin-Biosyntheseweges, des Cystein-Stoffwechselwegs, der Aspartatsemialdehyd-Synthese, der Glykolyse, der Anaplerotik, des Pentose-Phosphat-Stoffwechsels, des Zitronensäure-Zyklus oder des Aminosäure-Exports zu verstärken.

So kann für die Herstellung von Methionin, eines oder mehrere der folgenden Gene verstärkt sein:
- das für eine Aspartatkinase kodierende Gen lysC (EP 1 108 790 A2; DNA-SEQ NO. 281),
- das für eine Aspartat-Semialdehyd kodierende Gen asd (EP 1 108 790 A2; DNA-SEQ NO. 282),
- das für die Glycerinaldehyd-3-Phosphat Dehydrogenase kodierende Gen gap (Eikmanns (1992), Journal of Bacteriology 174: 6076-6086),
- das für die 3-Phosphoglycerat Kinase kodierende Gen pgk (Eikmanns (1992), Journal of Bacteriology 174: 6076-6086),
- das für die Pyruvat Carboxylase kodierende Gen pyc (Eikmanns (1992), Journal of Bacteriology 174: 6076-6086),
- das für die Triosephosphat Isomerase kodierende Gen tpi (Eikmanns (1992), Journal of Bacteriology 174: 6076-6086),
- das für die Homoserin O-Acetyltransferase kodierende Gen metA (EP 1 108 790 A2; DNA-SEQ NO. 725),
- das für die Cystathionin-gamma-Synthase kodierende Gen metB (EP 1 108 790 A2; DNA-SEQ NO. 3491),
- das für die Cystathionin-gamma-Lyase kodierende Gen metC (EP 1 108 790 A2; DNA-SEQ NO. 3061),
- das für die Cystathionin-Synthase kodierende Gen metH (EP 1 108 790 A2; DNA-SEQ NO. 1663),
- das für die Serin-Hydroxymethyltransferase kodierende Gen glyA (EP 1 108 790 A2; DNA-SEQ NO. 1110),
- das für die O-Acetylhomoserin-Sulfhydrylase kodierende Gen metY (EP 1 108 790 A2; DNA-SEQ NO. 726),
- das für die Methylentetrahydrofolat-Reduktase kodierende Gen metF (EP 1 108 790 A2; DNA-SEQ NO. 2379),
- das für die Phosphoserin-Aminotransferase kodierende Gen serC (EP 1 108 790 A2; DNA-SEQ NO. 928)
- eines für die Phosphoserin-Phosphatase kodierende Gen serB (EP 1 108 790 A2; DNA-SEQ NO. 334, DNA-SEQ NO. 467, DNA-SEQ NO. 2767)
- das für die Serine Acetyl-Transferase kodierende Gen cysE (EP 1 108 790 A2; DNA-SEQ NO. 2818)
- das für die Cystein-Synthase kodierende Gen cysK (EP 1 108 790 A2; DNA-SEQ NO. 2817),
- das für eine Homoserin-Dehydrogenase kodierende Gen hom (EP 1 108 790 A2; DNA-SEQ NO. 1306)

Zusätzlich kann es für die erfindungsgemäße Herstellung von Methionin vorteilhaft sein, gleichzeitig wenigstens eines der folgenden Gene so zu mutieren, dass die korrespondierenden Proteine, verglichen mit nicht mutierten Proteinen, in geringerem Maße oder nicht durch einen Stoffwechselmetaboliten in ihrer Aktivität beeinflusst werden oder dass ihre spezifische Aktivität gesteigert wird:
- das für eine Aspartatkinase kodierende Gen lysC (EP 1 108 790 A2; DNA-SEQ NO. 281),
- das für die Pyruvat Carboxylase kodierende Gen pyc (Eikmanns (1992), Journal of Bacteriology 174: 6076-6086),
- das für die Homoserin O-Acetyltransferase kodierende Gen metA (EP 1 108 790 A2; DNA-SEQ NO. 725),
- das für die Cystathionin-gamma-Synthase kodierende Gen metB (EP 1 108 790 A2; DNA-SEQ NO. 3491),
- das für die Cystathionin-gamma-Lyase kodierende Gen metC (EP 1 108 790 A2; DNA-SEQ NO. 3061),
- das für die Methionin-Synthase kodierende Gen metH (EP 1 108 790 A2; DNA-SEQ NO. 1663),
- das für die Serin-Hydroxymethyltransferase kodierende Gen glyA (EP 1 108 790 A2; DNA-SEQ NO. 1110),
- das für die O-Acetylhomoserin-Sulfhydrylase kodierende Gen metY (EP 1 108 790 A2; DNA-SEQ NO. 726),
- das für die Methylentetrahydrofolat-Reduktase kodierende Gen metF (EP 1 108 790 A2; DNA-SEQ NO. 2379),
- das für die Phosphoserin-Aminotransferase kodierende Gen serC (EP 1 108 790 A2; DNA-SEQ NO. 928)
- eines für die Phosphoserin-Phosphatase kodierende Gen serB (EP 1 108 790 A2; DNA-SEQ NO. 334, DNA-SEQ NO. 467, DNA-SEQ NO. 2767)
- das für die Serine Acetyl-Transferase kodierende Gen cysE (EP 1 108 790 A2; DNA-SEQ NO. 2818)
- das für die Cystein-Synthase kodierende Gen cysK (EP 1 108 790 A2; DNA-SEQ NO. 2817),
- das für eine Homoserin-Dehydrogenase kodierende Gen hom (EP 1 108 790 A2; DNA-SEQ NO. 1306)

Weiterhin kann es für die Produktion von Methionin vorteilhaft sein, eines oder mehrere der folgenden Gene abzuschwächen, insbesondere deren Expression zu verringern, oder auszuschalten:
- das für die S-Adenosylmethionin-Synthase (E.C.2.5.1.6) kodierende Gen metK
- das für die Homoserine-Kinase kodierende Gen thrB (EP 1 108 790 A2; DNA-SEQ NO. 3453)
- das für die Threonin Dehydratase kodierende Gen ilvA (EP 1 108 790 A2; DNA-SEQ NO. 2328)
- das für die Threonin Synthase kodierende Gen thrC (EP 1 108 790 A2; DNA-SEQ NO. 3486)
- das für die Meso-Diaminopimelat D-Dehydrogenase kodierende Gen ddh (EP 1 108 790 A2; DNA-SEQ NO. 3494)
- das für die Phosphoenolpyruvat-Carboxykinase kodierende Gen pck (EP 1 108 790 A2; DNA-SEQ NO. 3157)
- das für die Glucose-6-Phosphat-6-Isomerase kodierende Gen pgi (EP 1 108 790 A2; DNA-SEQ NO. 950)
- das für die Pyruvat-Oxidase kodierende Gen poxB (EP 1 108 790 A2; DNA-SEQ NO. 2873)
- das für die Dihydrodipicolinat Synthase kodiernde Gen dapA(EP 1 108 790 A2; DNA-SEQ NO. 3476)
- das für die Dihydrodipicolinat Reduktase kodiernde Gen dapB (EP 1 108 790 A2; DNA-SEQ NO. 3477)
- das für die Diaminopicolinat Decarboxylase kodiernde Gen lysA (EP 1 108 790 A2; DNA-SEQ NO. 3451)

Weiterhin kann es für die Produktion von Methionin vorteilhaft sein, wenigstens eines der oben genannten Gene metK, thrB, ilvA, thrC, ddh, pck, pgi, poxB, dapA, dapB, lysA so zu mutieren, dass die enzymatische Aktivität des korrespondierenden Proteins teilweise oder vollständig verringert wird.

Weiterhin kann es für die Produktion von Methionin vorteilhaft sein, weitere unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

Zur Erzielung einer Überexpression kann der Fachmann unterschiedliche Maßnahmen einzeln oder in Kombination ergreifen. So kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Methionin-Produktion zu steigern. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Biotechnology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der EP 0472869, in US 4,601,893, bei Schwarzer und Pühler (Biotechnology 9, 84-87 (1991), bei Remscheid et al. (Applied and Environmental Microbiology 60,126-132 (1994), bei LaBarre et al. (Journal of Bacteriology 175, 1001-1007 (1993)), in der WO 96/15246, bei Malumbres et al. (Gene 134, 15-24 (1993)), in der JP-A-10-229891, bei Jensen und Hammer (Biotechnology and Bioengineering 58, 191-195 (1998)), bei Makrides (Microbiological Reviews 60:512-538 (1996) und in bekannten Lehrbüchern der Genetik und Molekularbiologie.

### D) Durchführung der erfindungsgemäßen Fermentation

Die erfindungsgemäß hergestellten Mikroorganismen können kontinuierlich oder diskontinuierlich im batch Verfahren (Satzkultivierung) oder im fed batch (Zulaufverfahren) oder repeated fed batch Verfahren (repetitives Zulaufverfahren) zur Produktion von Methionin kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese erfindungsgemäß einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette, wie z. B. Sojaöl, Sonnenblumenöl, Erdnußöl und Kokosfett; Fettsäuren, wie z. B. Palmitinsäure, Stearinsäure oder Linolsäure; Alkohole, wie z. B. Glycerin, Methanol oder Ethanol; und organische Säuren, wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-,Phosphor- oder Sulfatsalze von Calcium, Magnesium, Natrium, Kobalt, Molybdän, Kalium, Mangan, Zink, Kupfer und Eisen.

Als Schwefelquelle für die Herstellung von Methionin, können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht läßt sich während der Anzucht durch Zugabe von basischen Verbindungen, wie Natriumhydroxid, Kaliumhydroxid, Ammoniak bzw. Ammoniakwasser, oder sauren Verbindungen, wie Phosphorsäure oder Schwefelsäure, kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff haltige Gasmischungen, wie z. B. Umgebungsluft, in die Kultur eingetragen. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen, Methionin enthaltenden, Fermentationsbrühen haben üblicherweise eine Trockenmasse von 7,5 bis 25 Gew.-%.

Vorteilhaft ist außerdem auch, wenn die Fermentation zumindest am Ende, insbesondere jedoch über mindestens 30% der Fermentationsdauer zuckerlimitiert gefahren wird. Das heißt, dass während dieser Zeit die Konzentration an verwertbarem Zucker im Fermentationsmedium auf ≥ 0 bis 3 g/l gehalten, beziehungsweise abgesenkt wird.

### E) Reinigung von Methionin

Sollte das erfindungsgemäß nach Auskristallisation erhaltene Methionin noch nicht die gewünschte Reinheit aufweisen, so kann dieses weiter aufgereinigt werden. Hierzu wird das Produkt in gelöster Form einer Chromatographie mit einem geeigneten Harz unterworfen, wobei das gewünschte Produkt oder die Verunreinigungen ganz oder teilweise auf dem Chromatographieharz zurückgehalten werden. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung bewandert. Das gereinigte Produkt kann durch Filtration oder Ultrafiltration konzentriert und bei einer Temperatur aufbewahrt werden, bei der die Stabilität des Produktes maximal ist.

Die Identität und Reinheit der isolierten Verbindung kann durch bekannte Techniken bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefasst in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

### F) Trocknung der Biomasse

Nach Beendigung der Fermentation kann die Methionin-haltige Fermentationsbrühe direkt zum fertigen trockenen Futtermitteladditiv verarbeitet werden. Gemäß einer bevorzugten Ausführungsform der Erfindung wird jedoch aus der Fermentationsbrühe zunächst der Biomasseanteil ganz oder teilweise, vorzugsweise vollständig, beispielsweise durch Zentrifugation, entfernt und zum erfindungsgemäßen Futtermitteladditiv verarbeitet. Die anfallende Biomasse enthält noch einen gewissen Anteil an Methionin, der gewünschtenfalls durch Zwischenschaltung eines Waschschritts verringert werden kann.

Die Aufarbeitung erfindungsgemäßer Biomasse zu einem geeigneten Trockenprodukt kann nach verschiedenen, aus dem Stand der Technik an sich bekannten Verfahren erfolgen. Insbesondere eignen sich zur Herstellung Trocknungsverfahren, wie Sprühtrocknung, Sprühgranulation, Kontakttrocknung, Wirbelschichttrocknung oder Gefriertrocknung. Geeignete Verfahren sind beispielsweise beschrieben in:
O. Krischer, W. Kast, Trocknungstechnik Erster Band, "Die wissenschaftlichen Grundlagen der Trocknungstechnik", Springer-Verlag 1978; Krischer/Kröll, Trocknungstechnik Zweiter Band, "Trockner und Trocknungsverfahren", Springer-Verlag 1959; K. Kröll, W. Kast, Trocknungstechnik Dritter Band, "Trocknen und Trockner in der Produktion", Springer-Verlag 1989; K. Masters, "Spray Drying Handbook", Longman Scientific & Technical 1991, 725 Seiten; H. Uhlemann, L. Mörl, "Wirbelschicht - Sprühgranulation", Springer-Verlag 2000; Gefriertrocknung: Georg-Wilhelm Oetjen, "Gefriertrocknen", VCH 1997; sowie EP-A-0 809 940. Auf die Offenbarung der oben beschriebenen Druckschriften wird hiermit ausdrücklich Bezug genommen.

Besonders bevorzugt erfolgt der erfindungsgemäße Trocknungsschritt durch Sprühtrocknung, wie beispielsweise Sprühtrocknung mit integriertem Wirbelbett, oder durch Sprühgranulation.

Falls erwünscht, kann die Trocknung in Gegenwart eines geeigneten futtermitteltauglichen Trägermaterials durchgeführt werden, wodurch insbesondere die Rieselfähigkeit und damit die Produktqualität verbessert werden kann.

Als futtermitteltaugliche Trägermaterialien können übliche inerte Träger verwendet werden. Ein "inerter" Träger darf keine negativen Wechselwirkungen mit den in dem Additiv enthaltenen Nahrungszusätzen zeigen und muss für die Verwendung als Hilfsstoff in Futtermittelzusätzen unbedenklich sein. Als Beispiele für geeignete Trägermaterialien sind zu nennen: anorganische oder organische Verbindungen natürlichen oder synthetischen Ursprungs. Beispiele für geeignete niedermolekulare anorganische Träger sind Salze, wie Natriumchlorid, Calciumcarbonat, Natriumsulfat und Magnesiumsulfat, oder Kieselsäure. Beispiele für geeignete organische Träger sind insbesondere Zucker, wie z. B. Glucose, Fructose, Saccharose sowie Dextrine und Stärkeprodukte. Als Beispiele für höhermolekulare organische Träger sind zu nennen: Stärke- und Cellulosepräparate, wie insbesondere Maisstärke, Getreidemehle, wie z. B. Weizen-, Roggen-, Gersten- und Hafermehl oder Gemische davon oder Weizengrießkleie. Das Trägermaterial kann in dem Präparat, bezogen auf Trockenbasis, in einem Anteil von etwa 5 bis 85 Gew.-%, wie z.B. etwa 10 bis 30 Gew.-%, 20 bis 40 Gew.-% oder 50 bis 85 Gew.-%, enthalten.

Im folgenden sollen einige bevorzugte Trocknungstechniken in allgemeiner Form kurz abgehandelt werden.

Die Sprühtrocknung kann so durchgeführt werden, dass man zunächst die noch feuchte Biomasse zum Zerstäuber im Sprühturm pumpt. Die Zerstäubung erfolgt z.B. mittels einer Druckdüse (Einstoffdüse), einer Zweistoffdüse oder eines Zentrifugalzerstäubers. Die Trocknung der Tröpfchen erfolgt durch einen in den Sprühtrockner geleiteten Heißluftstrom. Bei Verwendung von Zentrifugalzerstäubern erfolgt die Trocknung vorzugsweise im Gleichstrom. Bei Düsen kann die Trocknung auch im Gegen- oder Mischstrom erfolgen. Das getrocknete Pulver kann am Turm ausgetragen werden oder es wird mit dem Luftstrom mitgeführt und in einem Zyklon und/oder Filter abgetrennt. Je nach Produkt und Fahrweise kann eine Nachtrocknung erforderlich sein, die in einem internen, an den Sprühtrockner aufgeflanschten oder einem externen Wirbelbett erfolgen kann.

In einer Variante des erfindungsgemäßen Trocknungsverfahrens wird dem Trocknungsschritt, insbesondere der Sprühtrocknung eine kontinuierliche oder diskontinuierliche Wirbelbettagglomeration nachgeschaltet. Dazu wird in einem Wirbelbetttrockner zu Beginn des Verfahrens ein pulverförmiges Material, z.B. durch Sprühtrocknung erhaltenes pulverförmiges Additiv, vorgelegt. Die Verwirbelung erfolgt z.B. durch Zufuhr vorgewärmter Luft. Man sprüht auf die Wirbelschicht eine Flüssigphase, wie z.B. weitere Biomasse oder eine bindemittelhaltige Lösung, auf, wodurch man das vorgelegte Pulver mit dieser Lösung benetzt und durch deren Klebeeigenschaften zunehmend agglomeriert. Gleichzeitig wird kontinuierlich oder quasi-kontinuierlich, d.h. intervallweise getaktet, eine Teilmenge Agglomerat aus der Wirbelschicht ausgetragen. Der Austrag wird z.B. mit Hilfe eines Siebs klassiert. Dabei anfallendes Grobgut kann dabei gemahlen und kontinuierlich in das Wirbelbett wieder zurückgeführt werden. Feinanteile, wie z.B. aus der Abluftfilteranlage, können ebenfalls kontinuierlich zurückgeführt werden.

Eine weitere bevorzugte Verfahrensvariante umfasst eine Sprühtrocknung von Biomasse zu einem Pulver, gekoppelt mit der anschließenden Agglomeration des sprühgetrockneten Pulvers. Diese kann diskontinuierlich oder kontinuierlich durchgeführt werden. Bevorzugt ist die kontinuierliche Fahrweise. Derartige Verfahren können unter Verwendung herkömmlicher Sprühtrocknungsanlagen durchgeführt werden. Vorteilhafterweise erfolgt die Durchführung aber in Vorrichtungen, welche als FSD (Fluidized Spray Dryer) SBD (Spray Bed Dryer) oder MSD (Multi Stage Dryer) bekannt sind.

Eine Fluidized Spray Dryer (FSD)-Trocknungsanlage zur kontinuierlichen Herstellung eines erfindungsgemäßen Trockenprodukt kann insbesondere nach folgendem Schema betrieben werden: Feuchte Biomasse wird über eine Zuleitung in den Kopf des FSD-Trockners eingeleitet und mit Hilfe eines Zerstäubers zerstäubt. Die Trocknung erfolgt durch Einleitung von Luft im Gleichstrom. Die Luft wird dabei über eine Heizung vorgewärmt. Das sprühgetrocknete Pulver sammelt sich im integrierten Wirbelbett im Boden des FSD-Trockners und wird dort mit Hilfe einer Sprühvorrichtung unter Verwendung von Druckluft z.B. mit einer Bindemittellösung besprüht und mit eingeleiteter Luft verwirbelt. Die Luft wird dazu vorgewärmt und durch eine Zuleitung unterhalb des Anströmbodens des integrierten Wirbelbettes zugeführt. Das dabei anfallende Voragglomerat gelangt anschließend in ein nachgeschaltetes, externes Wirbelbett. In dieses externe Wirbelbett wird von unten über eine weitere Zuleitung vorgewärmte Luft eingeführt. Das im Wirbelbett vorgelegte Voragglomerat wird mit Hilfe einer weiteren Sprühvorrichtung unter Verwendung von Druckluft erneut besprüht (z.B. mit Bindemittellösung) und zum Endprodukt agglomeriert. Das fertige Agglomerat wird aus dem Wirbelbett ausgetragen und kann, wie oben beschrieben, weiter aufgearbeitet werden.

Die Zusammensetzung und Menge der eingedüsten Flüssigkeiten richten sich nach den Klebeeigenschaften der eingesprühten Lösung, der zu erzielenden Agglomeratgrösse und den Prozessbedingungen.

Für den Fall, dass die Klebeeigenschaften der aufgesprühten Biomasse nicht ausreichen, um nach dem Aufsprühen ein stabiles Verkleben der Partikel zu gewährleisten, ist zusätzlich die Verwendung eines Bindemittels von Vorteil. Dadurch wird vermieden, dass die Agglomerate beim Trocknen wieder zerfallen. In solchen Fällen ist es bevorzugt, ein in wässrigem Medium lösliches oder dispergierbares Bindemittel in das Wirbelbett einzusprühen. Als Beispiele für geeignete Bindemittel sind zu nennen Lösungen von Kohlehydraten, wie z.B. Glucose, Saccharaose, Dextrine u.a., Zuckeralkohole, wie z.B. Mannit, oder Polymerlösungen, wie beispielsweise Lösungen von Hydroxypropylmethylcellulose (HPMC), Polyvinylpyrrolidon (PVP), ethoxylierte Cellulose (EC), Ethylcellulose oder Propylcellulose. Durch gezielte Wahl von Menge und Klebeeigenschaften des eingesprühten Bindemittels entstehen Agglomerate unterschiedlicher Größe und Festigkeit.

Wird das Bindemittel als separate Lösung aufgesprüht, so liegt der Bindemittelanteil der Lösung im Bereich von etwa 1 bis 30 Gew.-%, bezogen auf das Gesamtgewicht der Lösung. Das Bindemittel liegt hierbei ebenfalls gelöst in einem wässrigen Medium, vorzugsweise keimfreies, entsalztes Wasser, vor. Übliche Zusätze, wie z.B. Puffer, oder Lösungsvermittler, können ebenfalls enthalten sein.

Der Anteil des Bindemittels im Endprodukt beträgt erfindungsgemäß 0 bis etwa 20 Gew.-%, beispielsweise etwa 1 bis 6 Gew.-%. Die optimale Menge ist auch von der Art des gewählten Bindemittels abhängig. Dabei ist darauf zu achten, dass negative Einflüsse auf das Produkt vermieden werden.

### G) Formulierungen

### i) Futtermitteladditive und Futtermittelzusammensetzungen:

Das Methionin-haltige Futtermitteladditiv liegt vorzugsweise als feinteiliges, rieselfähiges Pulver bzw. in granulierter Form vor. Die Partikel können da- bei z.B. in einem Größenbereich von sind 5 bis 200 [µm, wie z.B. 10 bis 150 µm, 20 bis 100µm oder 30 bis 80 µm, liegen, ohne darauf beschränkt zu sein.

Die Schüttdichte der erfindungsgemäßen Additive kann z.B. im Bereich von etwa 100 bis 600 g/l, wie z.B. 150 bis 400 g/l oder 200 bis 350 g/l, liegen, ohne darauf beschränkt zu sein.

Der Methionin-Gehalt des erfindungsgemäßen Additivs variiert je nach Herstellungsweise.

Erfindungsgemäß erhältliches Methioninkristallisat weist dabei einen Methioningehalt von mehr als 60 Gew.-%, wie z.B. etwa 70 bis 98 Gew.-%, vorzugsweise etwa 80 bis 95 Gew.-%, besonders bevorzugt etwa 87 bis 95 Gew.-% auf. Der Anteil von Salzen (Rückstande aus der Fermentationsbrühe) kann dabei im Bereich von etwa 0 bis 20 Gew.-%, insbesondere etwa 5 bis 15 Gew.-% liegen. Sonstige Fermentationsnebenbestandteile können in einem Anteil von etwa 0 bis 20 Gew.-%, insbesondere etwa 5 bis 15 Gew.-%, enthalten sein.

Erfindungsgemäß hergestelltes Biomassemethionin weist dabei einen Methioningehalt von mehr als 3 Gew.-%, wie z.B. etwa 5 bis 40 Gew.-%, oder etwa 10 bis 35 Gew.-%, auf. Der Anteil von Salzen kann dabei im Bereich von etwa 0 bis 30 Gew.-%, wie etwa 5 bis 25 Gew.-% liegen. Sonstige Fermentationsnebenbestandteile können in einem Anteil von etwa 0 bis 20 Gew.-%, wie etwa 5 bis 15 Gew.-%, enthalten sein.

Der Restfeuchtegehalt des fertigen Additivs liegt vorzugsweise im Bereich von weniger als etwa 3-5 Gew.-%, bezogen auf das Gesamtgewicht des Additivs. Obige Gewichtsprozentangaben sind bezogen auf das Gesamtgewicht des Trockenprodukts (vorzugs-40 weise ohne Restfeuchte).

Neben den oben beschriebenen Bestandteilen können die Formu- lierungen, wie oben bereits erwähnt, weitere Zusätze enthalten, die vor, während oder nach Aufarbeitung der Biomasse zugesetzt werden können. Als Beispiele können genannt werden Konservierungsstoffe, Antibiotika, antimikrobielle Zusätze, Antioxidantien, Chelatbildner, physiologisch unbedenkliche Salze, Geschmacksstoffe, Farbstoffe und dergleichen. Auch ernährungsrelevante Zusätze können enthalten sein, wie z.B. Vitamine, (z.B. die Vitamine A, B1, B_{2,} B_{6,} B₁₂. C, D₃, und/oder E, K₃. Folsäure, Nicotinsäure, Pantothensäure); Taurin, Garbonsäuren und deren Salze, wie z.B. Tricarbonsäuren, wie Citrat, Isocitrat, trans-/cis-Aconitat, und/oder homo-Citrat, Enzyme, Carotinoide, Mineralien, wie z.B. P, Ca, Mg und/oder Fe, und Spurenelemente, wie Se, Cr, Zn, Mn, Proteine, Kohlenhydrate, Fette, Aminosäuren. Weiterhin können Brenztraubensäure, L-Carnitin, Liponsäure, Coenzym Q10, Aminocarbonsäuren, wie z.B. Kreatin, Orotsäure, Myoinositol, Flavonoide, Betain, p-Aminobenzoesäure, enthalten sein.

Die Methionin enthaltenden Futtermitteladditive können in han- delsübliche Tierfutterformulierungen eingearbeitet werden, welche beispielsweise dann an Rinder, Schweine, Schafe, Geflügel und dergleichen verfüttert werden können.
Hierzu wird das erfindungsgemäße Additiv mit üblichen Tierfutterbestandteilen vermischt und gegebenenfalls konfektioniert, beispielsweise pelletiert. Übliche Tierfutterbestandteile sind z.B. Mais, Gerste, Maniok, Hafer, Soja, Fischmehl, Weizengrießkleie, Sojaöl, Kalk, Mineralien, Spurenelemente, Aminosäuren und Vitamine.

### ii) Nahrungs- und Futterergänzungsmittel

Das erfindungsgemäß hergestellte Methionin findet Verwendung als Zusatz in Nahrungs- und Futtermitteln oder Zusatz in Nahrungs- und Futterergänzungsmitteln, wie z.B. Multivitaminpräparaten. Das erfindungsgemäß hergestellte Produkt kann dazu in der gewünschten Menge und in an sich bekannter Weise in herkömmliche Nahrungs- und Futtermittel bzw. Nahrungs- und Futterergänzungsmitteln eingearbeitet werden. Es kann dabei je nach Verwendung in den unterschiedlichen, zweckmäßigen Anteilen enthalten sein.

### iii) Beschichtete Formulierungen

Die oben beschriebenen Formulierungen können gegebenenfalls zusätzlich ein Coating aufweisen. Sie sind dabei mit einem Beschichtungsmittel verse- hen, welches wenigstens eine Verbindung, ausgewählt unter:
- Polyalkylenglycolen, insbesondere Polyethylenglycolen, beispeilsweise mit einem zahlenmittleren Molekulargewicht von etwas 400 bis 15 000, wie z.B. 400 bis 10 000;
- Polyalkylenoxid-Polymeren oder -Copolymeren, beispielsweise mit einem zahlenmäßigen Molekulargewicht von etwa 4000 bis 20 000, insbesondere Blockcopolymeren von Polyoxyethylen und Polyoxypropylen;
- substituierten Polystyrolen, Maleinsäurederivaten und Styrol-Maleinsäurecopolymeren;
- Vinylpolymeren, insbesondere Polyvinylpyrrolidonen, beispielsweise mit einem zahlenmittleren Molekulargewicht von etwas 7 000 bis 1 000 000; entweder alleine oder in Kombination mit anderen Verbindungen, wie Celluloseethern oder Stärken;
- Vinylpyrrolidon/Vinylacetat-Copolymeren, beispielsweise mit einem zahlenmittleren Molekulargewicht von etwa 30 000 bis 100 000;
- Polyvinylalkoholen, beispielsweise mit einem zahlenmittleren Molekulargewicht von etwa 10 000 bis 200000, und Polyphthalsäurevinylestern;
- Hyroxypropylmethylcellulosen, beispielsweise mit einem zahlenmittleren Molekulargewicht von etwa 6000 bis 80 000;
- Alkyl(meth)acrylat-Polymeren und -Copolymeren, beispielsweise mit einem zahlenmittleren Molekulargewicht von etwa 100 000 bis 1 000 000, insbesondere Ethylacrylat/Methylmethacrylat-Copolymeren und Methacrylat/Ethylacrylat-Copolymeren;
- Polyvinylacetaten, beispielsweise mit einem zahlenmittleren Molekulargewicht von etwas 250 000 bis 700 000 gegebenenfalls stabilisiert mit Polyvinylpyrrolidon;
- Polyalkylenen, insbesonderen Polyethylenen;
- Aromatischen Polymeren, beispielsweise Ligninen;
- Polyacrylsäuren;
- Polyacrylamiden;
- Polycyanoacrylaten;
- Phenoxyessigsäure-Formaldehyd-Harzen;
- Cellulosederivaten, wie Ethylcellulose, Ethylmethylcellulose, Methylcellulose, Hydroxypropylcellulose, Hydroxypropylmethlycellulose, Carboxymethylcellulose, Celluloseacetatphthalat;
- tierischen, pflanzlichen oder synthetischen Fetten und modifizierten Fetten, wie beispielsweise Polyglycolen, Fettalkoholen, ethoxylierten Fettalkoholen, höheren Fettsäuren; Mono-, Di- and Triglyceriden von höheren Fettsäuren, z.B. Glycerinmonosterat, Alkylarylethoxylate und Kokosmonoethanolamide;
- tierischen und pflanzlichen Wachsen oder chemisch modifizierten tierischen und pflanzlichen Wachsen, wie Bienenwachs, Candelillawachs, Carnaubawachs, Montanesterwachs und Reiskeimölwachs, Walrat, Lanolin, Jojobawachs, Sasolwachs;
- tierischen und pflanzlichen Proteinen, wie z.B. Gelatine, Gelatinederivate, Gelatineersatzstoffe, Casein, Molke, Keratin, Sojaprotein; Zein und Weizenprotein;
- Mono- und Disacchariden, Oligosacchariden, Polysacchariden, wie z.B. Hyaluronsäure, Pullulan, Elsinan, Stärken, modifizierten Stärken, sowie Pektinen, Alginaten, Chitosan, Carrageen;
- pflanzlichen Ölen, wie z.B. Sonnenblumen-, Distel-, Baumwollsaat-, Soja-, Maiskeim-, Oliven-, Raps-, Lein-, Ölbaum-, Kokos-, Ölpalmkernöl; synthetischen oder halbsynthetischen Ölen, wie z.B. mittelkettigen Triglyceriden oder Mineralölen; tierischen Ölen, wie z.B. Hering-, Sardine- und Walöl;
- gehärteten (hydrierten oder teilhydrierten) Ölen/Fetten, wie z.B. von den oben genannten, insbesondere hydriertes Palmöl, hydriertes Baumwollsaatöl, hydriertes Sojaöl;
- Lackcoatings, wie z.B. Terpenen, insbesondere Schellack, Tolubalsam, Perubalsam, Sandarak, und Silikonharzen;
- Fettsäuren, sowohl gesättigte als auch einfach und mehrfach ungesättigte C₆ bis C₂₄-Carbonsäuren;
- Kieselsäuren;
und Mischungen davon umfasst.

Die Zugabe von Weichmachern oder Emulgatoren zu Fetten oder Wachsen vor der Beschichtung kann gegebenenfalls zur Verbesserung der Flexibilität des Films vorteilhaft sein.

Das Aufbringen von Beschichtungen erfolgt in an sich bekannter Weise, gegebenenfalls zusammen mit Additiven, in der Regel über Einrichtungen zum Auftropfen oder Aufdüsen auf das in einem Mischer vorgelegte Wertprodukt. Beispiele hierfür sind Lanzen, Brauseköpfe, Einstoff- oder Mehrstoffdüsen, oder rotierende Tropf- oder Zerstäubungseinrichtungen. Im einfachsten Fall ist die Zugabe auch lokal als konzentrierter Strahl möglich. Alternativ kann im Mischer zunächst das Coatingmaterial vorgelegt werden, um danach das Wertprodukt zuzugeben. Eine weitere Möglichkeit besteht in der Zugabe von zunächst festem Coatingmaterial, welches infolge der Wandheizung oder aufgrund von mechanischem Energieeintrag schmilzt und das Wertprodukt überzieht.

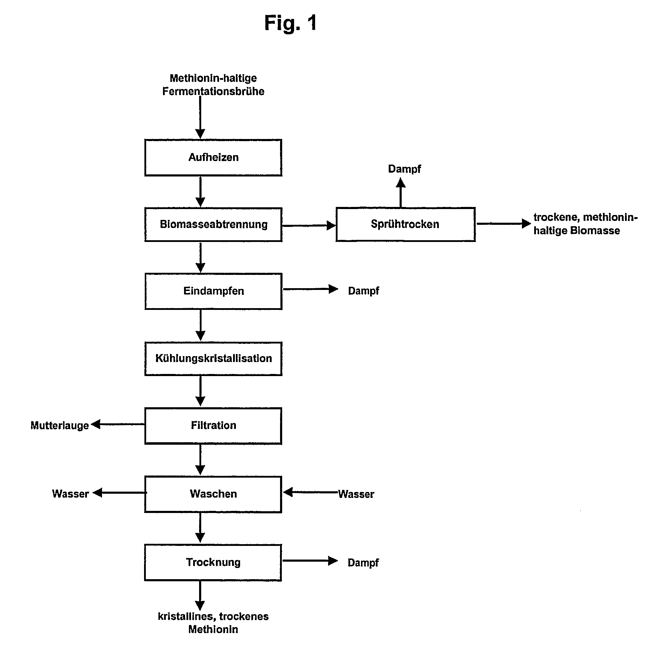

Die Erfindung wird nun unter Bezugnahme auf beiliegende Figuren näher beschrieben. Dabei zeigen die Figuren 1 bis 4 verschiedene Ausgestaltungen eines erfindungsgemäßen Verfahrens zur Herstellung von kristallinem, trockenen Methionin und trockener, methioninhaltiger Biomasse ("Biomassemethionin").

### Beispiel 1:

### a) Fermentative Herstellung vo Methionin

Für die Herstellung einer repräsentativen Fermentationsbrühe für die Reinigung von Methionin wurde eine Labor-Fermentation durchgeführt. Der *Corynebacterium glutamicum* Stamm ATCC13032 (American Type Culture Collection, Manassas, USA) wurde in einer Vorkultur von 200 ml BHI Medium (Difco/Becton Dickinson Franklin Lakes, USA) angezogen. Im Techfors Fermenter wurde die Vorkultur dann in das Kulturmedium (ca.14 l) überimpft.

Das Fermentationsmedium der Hauptkultur war wie folgt zusammengesetzt
2 g/l KH₂PO₄
2 g/l K₂HPO₄
10 g/l Ammoniumsulfat
100 g/l Glucose
5 g/l Hefeextrakt
20 mg/l Kanamyzin
1 g/l KS911 ASM Antischaum
pH 7,0
mit vollentsalztem Wasser auffüllen auf gewünschtes Endvolumen

**Spurensalzlösung 1 ml/l Medium**

| | |
|---|---|
| FeSO₄ x 7 H₂O | 10 g/l |
| MnSO₄ x 4 - 6H₂O | 10 g/l |
| ZnSO₄ | 2 g/l |
| MgSO₄ x 7 H₂O | 250 g/l |
| Mit HCl auf pH 1 einstellen | |

1 ml/l Protokatechuat Lösung (Stammlösung 300 mg/10 ml)

| | |
|---|---|
| Biotin | 1 mg/l |
| Thiamin | 1 mg/l |
| CaCl₂ | 5 mg/l |

Nach Inokulation des Fermenters durch die Vorkultur wurde der Fermenter durch Zugabe von Base (25 % NH₄OH) auf pH 7 gehalten und fermentiert, bis der Zucker verbraucht war. Dies wurde durch einen Anstieg des pO2-Werts bzw. durch den Abfall von OTR und CTR angezeigt.

### b) Aufarbeitung der Fermentationsbrühe

Das Vorgehen bei der Aufarbeitung ist in Figur 1 schematisch skizziert.

Als Ausgangsstoff dient eine gemäß Abschnitt a) hergestellte Fermentationsbrühe. Bei einer Fermentationstemperatur zwischen 30 und 40 °C liegt ca. 50 % des enthaltenen Methionins in kristalliner Form vor. Das Ausgangsprodukt besitzt einen Wassergehalt von ca. 86 %, einen aufgestockten Methioningehalt von ca. 9 % und einen Biomassegehalt von ca. 3 %. Sonstige Fermentationsnebenprodukte und Mineralstoffe sind in Spuren (c.a. 2,5 Gew.-%) in der Fermentationsbrühe enthalten.

20 kg dieser Fermentationsbrühe werden 15 Minuten auf 70°C erwärmt. Das Methionin geht dadurch komplett in Lösung. Bei konstanter Temperatur wird dann die Biomasse abzentrifugiert. Der Überstand (ca. 15 kg) wird anschließend bei 100°C und Normaldruck auf einen Methioningehalt von 20 % aufkonzentriert. Das Konzentrat wird dann mit 5 K/h auf 5°C abgekühlt, wodurch ein Großteil des Methionins auskristallisiert. Die Kristalle werden dann auf einem Nutschenfilter aus der Kristallmaische abgetrennt, mit 4 Liter vortemperiertem Wasser (5°C) gewaschen und anschließend mit Stickstoff bei 40°C trockengeblasen. Durch dieses Vorgehen konnte 1,3 kg trockenes Methioin mit einer Reinheit ca 90 % isoliert werden.

Der Rückstand der Zentrifugation (ca. 5 kg) enthält neben Biotrockenmasse noch ca. 6 % Methionin. Durch Sprühtrocknung kann dieser Rückstand in ca. 0,7 kg leicht gelbliches und rieselfähiges Trockenpulver mit einer Restfeuchte von 3 % überführt werden, welches neben der Biotrockenmasse und sonstigen Fermentationsnebenprodukten und Mineralsalzen noch Methionin (ca. 30 %) enthält.

Die Sprühtrocknung erfolgte in einem Labor-Sprühtrockner mit folgenden Einstellungen des Geräts:
Eingangstemperatur: 200 °C,
Ausgangstemperatur: 80 - 82 °C.

Als Heizgas wurden 60 m³/h Stickstoff eingesetzt. Das Düsengas wurde mit einem Druck von 2 bar durch eine 1,2 mm Düse versprüht.

### Beispiel 2:

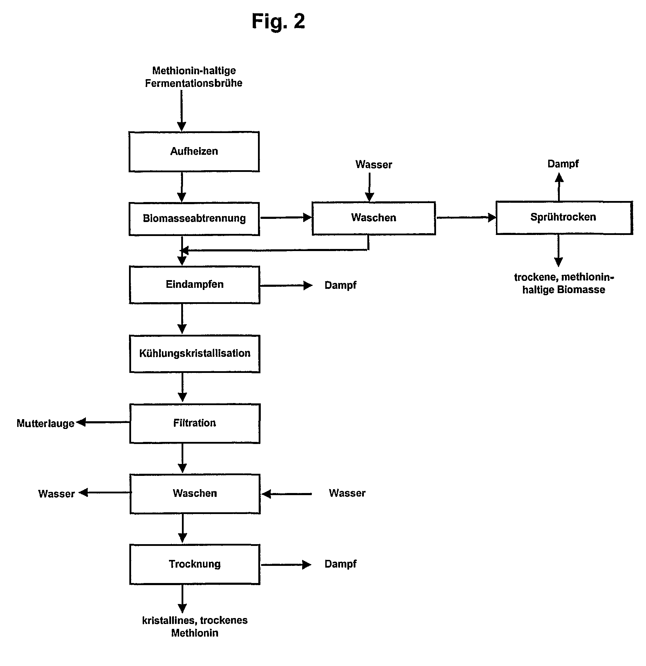

Ausgehend vom gleichen Ausgangsmaterial wird das Verfahren dahingehend modifiziert, dass die Biomasse mittels Zentrifugation abgetrennt und die abgetrennte Biomasse anschließend mit 5 l Wasser gewaschen wird (Figur 2). Nach Zentrifugation wird der entstandene Überstand zum Überstand der ersten Biomasseabtrennung gegeben. Der gesamte Überstand wird bei 100°C und Normaldruck auf einen Methioningehalt von 16 % aufkonzentriert. Durch Abkühlen des Konzentrates mit 5 K/h auf 5°C wird das Methionin auskristallisiert. Die Kristalle werden auf einem Nutschenfilter abgetrennt, mit 4,5 Liter vortemperiertem Wasser (5°C) gewaschen und anschließend mit Stickstoff bei 40 °C getrocknet. Durch dieses Vorgehen erhöht sich die Menge an trockenem Methionin auf ca. 1,5 kg. Die Reinheit des isolierten Kristallisates ist weiterhin ca. 90 %.

Der Rückstand der abgetrennten und gewaschenen Biomasse wird durch Sprühtrocknung in ca. 0,5 kg trockenes Produkt überführt

Das so hergestellte Produkt, welches neben Biotrockenmasse und sonstigen Fermentationsnebenprodukten und Mineralsalzen noch Methionin (ca. 10 %) enthielt, war rieselfähig.

### Beispiel 3:

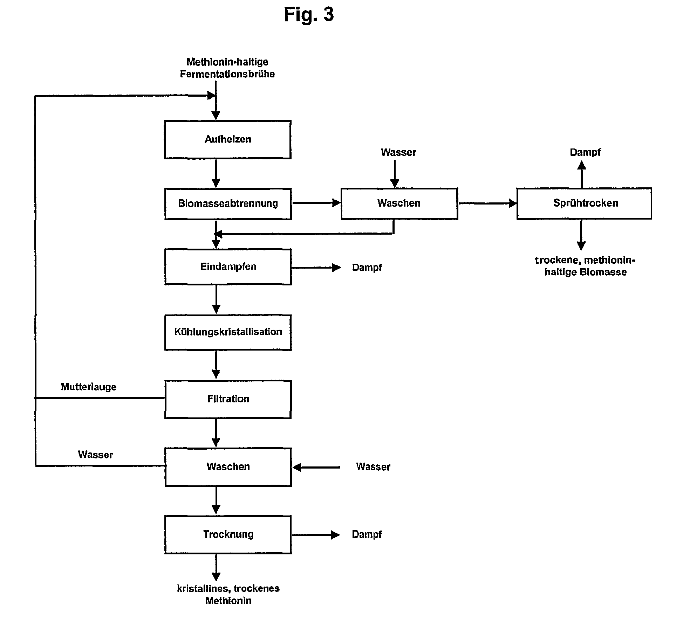

Ausgehend vom gleichen Ausgangsmaterial wird das Verfahren aus Beispiel 2 zusätzlich dahingehend modifiziert, dass die Mutterlauge und das Waschwasser, welche bei der Abtrennung des kristallinen Methionins anfallen, in einem nächsten Ansatz wieder der Methionin haltigen Fermentationsbrühe zugegeben werden (Figur 3).

Bei ansonsten analogem Vorgehen zu Beispiel 2 werden bei der Fermentation ca. 1,5 kg trockenes Methionin aus der Kristallisation mit einer Reinheit von ca. 90 % erhalten und ca. 0,5 kg Produkt aus der sprühgetrockneten Biomasse mit einem Methioningehalt von ca. 10 %.

### Beispiel 4:

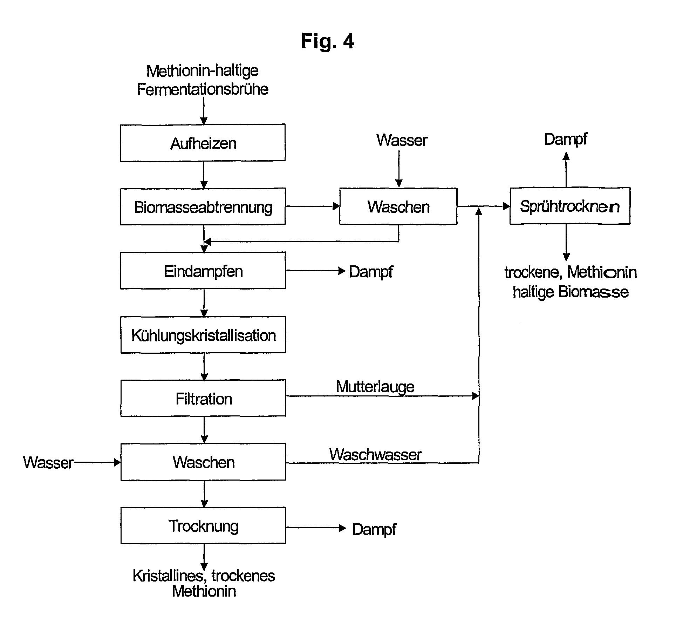

Ausgehend vom gleichen Ausgangsmaterial wird das Verfahren aus Beispiel 2 zusätzlich dahingehend modifiziert, dass die Mutterlauge und das Waschwasser, welche bei der Abtrennung des kristallinen Methionins anfallen, dem Biomassenstrom vor der Sprühtrocknung zugegeben werden (Figur 4).

Bei ansonsten analogem Vorgehen zu Beispiel 2 fällt ca. 1,1 kg Methionin-haltige Biomasse an, welche neben Biotrockenmasse und sonstigen Fermentationsnebenprodukten und Mineralsalzen noch Methionin (ca. 30 %) enthält. Die Menge an trockenem Methionin beträgt 1,5 kg mit einer Reinheit von ca. 90 %.

### Beispiel 5:

Es ist auch eine Verfahrensvariante denkbar, bei der ein Teil der Mutterlauge und des Waschwassers nach der Kristallisation des Methionins der Fermenterbrühe vor Biomassenabtrennung zugegeben wird und der andere Teilstrom dem Biomassenstrom vor der Sprühtrocknung (Kombination aus Beispiel 3 und 4).

## Patentansprüche

1. Verfahren zur Isolierung von fermentativ hergestelltem Methionin, wobei man
a) eine Methionin-haltige, bei der Fermentation eines Methionin-produzierenden Mikroorganismus anfallende Biomasse-haltige Fermentationsbrühe, auf eine Temperatur im Bereich von 60 bis 120 °C erwärmt, die ausreicht, um die Löslichkeit von Methionin in der Flüssigphase zu erhöhen,
b) daraus eine an Methionin angereicherte Flüssigphase gewinnt, indem man aus der angereicherten Fermentationsbrühe die Biomasse abtrennt und
c) Methionin, gegebenenfalls nach Aufkonzentrieren der angereicherten Flüssigphase, auskristallisiert;
wobei man die in Stufe b) abgetrennte Biomasse
g1) gegebenenfalls wäscht, wobei die zum Waschen verwendete Flüssigkeit gegebenenfalls erwärmt ist, und
g3) trocknet.

2. Verfahren nach Anspruch 1, wobei man in Stufe a) Bedingungen wählt, die ausreichen, um Methionin im Wesentlichen vollständig in Lösung zu bringen.

3. Verfahren nach einem der vorhergehenden Ansprüche, wobei man auf eine Temperatur im Bereich von 70 bis 100 °C erwärmt.

4. Verfahren nach einem der vorhergehenden Ansprüche, wobei man
d) das auskristallisierte Methionin abtrennt,
e) das abgetrennte feste Methionin gegebenenfalls wäscht und
f) gegebenenfalls trocknet.

5. Verfahren nach Anspruch 1, wobei man
g2) die in Stufe g1) anfallende Waschflüssigkeit mit der an Methionin angereicherten Flüssigphase von Stufe b) vereinigt.

6. Verfahren nach einem der Ansprüche 1 bis 5, wobei man die in Stufe d) anfallende Mutterlauge
d1) mit der Methionin-haltigen flüssigen Fraktion aus einem anderen Fermentationsansatz mit einem Methionin-produzierenden Mikroorganismus vereinigt; oder
d2) der abgetrennten Biomasse aus dem gleichen oder einem anderen Fermentationsansatz mit einem Methionin-produzierenden Mikroorganismus vor dem Trocknen gemäß Stufe g3) zusetzt.

7. Verfahren nach einem der Ansprüche 1 bis 6, wobei man die in Stufe e) anfallende Waschflüssigkeit
e1) mit der Methionin-haltigen flüssigen Fraktion aus einem anderen Fermentationsansatz mit einem Methionin-produzierenden Mikroorganismus vereinigt; oder
e2) der abgetrennten Biomasse aus dem gleichen oder einem .anderen Fermentationsansatz mit einem Methionin-produzierenden Mikroorganismus vor dem Trocknen gemäß Stufe g3) zusetzt.

8. Verfahren nach einem der Ansprüche 1 bis 7, wobei die Trocknung gemäß Stufe g3) einen Sprühtrocknungsschritt umfasst.

9. Verfahren zur fermentativen Herstellung von Methionin, wobei man einen natürlichen oder rekombinanten Mikroorganismus fermentiert und das gebildete Methionin nach einem Verfahren gemäß einem der vorhergehenden Ansprüche isoliert.

10. Verfahren nach einem der vorhergehenden Ansprüche, wobei der Methioninproduzierende Mikroorganismus ausgewählt ist unter natürlichen oder rekombinanten Bakterien der Gattung Corynebacterium.

11. Verfahren nach einem der vorhergehenden Ansprüche, wobei L-Methionin isoliert wird.

12. Verfahren nach einem der Ansprüche 1 bis 8, wobei das Trockengut der Stufe g3) in einem weiteren Schritt zur Herstellung eines Futtermittels oder eines Futterergänzungsmittels eingesetzt wird.

13. Verfahren nach einem der Ansprüche 1 bis 11, wobei man das isolierte Methionin in einem weiteren Schritt zur Herstellung eines L-Methionin enthaltenden Nahrungs- oder Futtermittels oder Nahrungs- oder Futtermittelergänzungsmittels einsetzt.

14. Verfahren nach einem der Ansprüche 1 bis 8, wobei man Biomasse und Methionin in einem weiteren Schritt zur Herstellung eines Feedadditivs einsetzt.

15. Verfahren nach Anspruch 14, wobei man in einem weiteren Schritt das Feedadditiv zur Herstellung einer Futtermittelzusammensetzung einsetzt.

## Claims

1. A process for isolating methionine produced by fermentation, which comprises
a) heating a methionine-containing biomass-containing fermentation broth produced in the fermentation of a methionine-producing microorganism to a temperature of from 60 to 120°C which is sufficient to increase the solubility of methionine in the liquid phase,
b) obtaining therefrom a methionine-enriched liquid phase, the biomass being separated off from the enriched fermentation broth and
c) crystallizing out methionine, if appropriate after concentrating the enriched liquid phase;
the biomass separated off in stage b) being
g1) if appropriate washed, the liquid used for washing being heated if appropriate, and
g3) dried.

2. The process according to claim 1, wherein, in stage a), conditions are chosen which are sufficient to bring methionine essentially completely into solution.

3. The process according to one of the preceding claims, wherein heating is performed to a temperature of from 70 to 100°C.

4. A process according to one of the preceding claims, wherein
d) the crystallized methionine is separated off,
e) the solid methionine which has been separated off is if appropriate washed and
f) if appropriate dried.

5. The process according to claim 1, wherein
g2) the wash liquid produced in stage g1) is combined with the methionine-enriched liquid phase from stage b).

6. The process according to one of claims 1 to 5, wherein the mother liquor produced in stage d) is
d1) combined with the methionine-containing liquid fraction from another fermentation batch using a methionine-producing microorganism; or
d2) added to the biomass separated off from the same or another fermentation batch using a methionine-producing microorganism before the drying according to stage g3).

7. The process according to one of claims 1 to 6, wherein the wash liquid produced in stage e) is e1
) combined with the methionine-containing liquid fraction from another fermentation batch using a methionine-producing microorganism; or
e2)) added to the biomass separated off from the same or another fermentation batch using a methionine-producing microorganism before the drying according to stage g3).

8. The process according to one of claims 1 to 7, wherein the drying according to stage g3) comprises a spray-drying step.

9. A process for producing methionine by fermentation, a natural or recombinant microorganism being fermented and the methionine formed being isolated by a process according to one of the preceding claims.

10. The process according to one of the preceding claims, wherein the methionine-producing microorganism is selected from natural or recombinant bacteria of the genus Corynebacterium.

11. The process according to one of the preceding claims, wherein L-methionine is isolated.

12. The process according to one of claims 1 to 8, wherein the dry material of stage g3) is used in a further step for producing a feedstuff or a feed supplement.

13. The process according to one of claims 1 to 11, wherein the methionine isolated is used in a further step for producing an L-methionine-containing foodstuff or feedstuff or food supplement or feed supplement.

14. The process according to one of claims 1 to 8, wherein biomass and methionine are used in a further step for producing a feed additive.

15. The process according to claim 14, wherein in a further step the feed additive is used for producing a feedstuff composition.

## Revendications

1. Procédé d'isolement de méthionine fabriquée par fermentation, dans lequel
a) un bouillon de fermentation contenant une biomasse formée lors de la fermentation d'un microorganisme produisant de la méthionine, contenant de la méthionine, est porté à une température dans la plage allant de 60 à 120 °C, qui est suffisante pour augmenter la solubilité de la méthionine dans la phase liquide,
b) une phase liquide enrichie en méthionine est obtenue à partir de celui-ci par séparation de la biomasse du bouillon de fermentation enrichi, et
c) la méthionine est cristallisée, éventuellement après concentration de la phase liquide enrichie ;
dans lequel la biomasse séparée à l'étape b)
g1) est éventuellement lavée, le liquide utilisé pour le lavage étant éventuellement chauffé, et
g3) séchée.

2. Procédé selon la revendication 1, dans lequel des conditions qui sont suffisantes pour solubiliser la méthionine essentiellement en totalité sont choisies à l'étape a).

3. Procédé selon l'une quelconque des revendications précédentes, dans lequel un chauffage est effectué à une température dans la plage allant de 70 à 100 °C.

4. Procédé selon l'une quelconque des revendications précédentes, dans lequel
d) la méthionine cristallisée est séparée,
e) la méthionine solide séparée est éventuellement lavée et
f) éventuellement séchée.

5. Procédé selon la revendication 1, dans lequel g2) le liquide de lavage formé à l'étape g1) est réuni avec la phase liquide enrichie en méthionine de l'étape b).

6. Procédé selon l'une quelconque des revendications 1 à 5, dans lequel la liqueur mère formée à l'étape d)
d1) est réunie avec la fraction liquide contenant de la méthionine issue d'une autre préparation de fermentation avec un microorganisme produisant de la méthionine ; ou
d2) est ajoutée à la biomasse séparée issue de la même ou d'une autre préparation de fermentation avec un microorganisme produisant de la méthionine avant le séchage selon l'étape g3).

7. Procédé selon l'une quelconque des revendications 1 à 6, dans lequel le liquide de lavage formé à l'étape e)
e1) est réuni avec la fraction liquide contenant de la méthionine issue d'une autre préparation de fermentation avec un microorganisme produisant de la méthionine ; ou
e2) est ajouté à la biomasse séparée issue de la même ou d'une autre préparation de fermentation avec un microorganisme produisant de la méthionine avant le séchage selon l'étape g3).

8. Procédé selon l'une quelconque des revendications 1 à 7, dans lequel le séchage selon l'étape g3) comprend une étape de séchage par pulvérisation.

9. Procédé de fabrication par fermentation de méthionine, dans lequel un microorganisme naturel ou recombinant est fermenté et la méthionine formée est isolée par un procédé selon l'une quelconque des revendications précédentes.

10. Procédé selon l'une quelconque des revendications précédentes, dans lequel le microorganisme produisant de la méthionine est choisi parmi les bactéries naturelles ou recombinantes du genre Corynebacterium.

11. Procédé selon l'une quelconque des revendications précédentes, dans lequel la L-méthionine est isolée.

12. Procédé selon l'une quelconque des revendications 1 à 8, dans lequel le produit séché de l' étape g3) est utilisé dans une étape ultérieure pour la fabrication d'un aliment pour animaux ou d'un complément alimentaire pour animaux.

13. Procédé selon l'une quelconque des revendications 1 à 11, dans lequel la méthionine isolée est utilisée dans une étape ultérieure pour la fabrication d'un aliment ou d'un aliment pour animaux ou d'un complément alimentaire ou d'un complément alimentaire pour animaux contenant de la L-méthionine.

14. Procédé selon l'une quelconque des revendications 1 à 8, dans lequel la biomasse et la méthionine sont utilisées dans une étape ultérieure pour la fabrication d'un additif alimentaire.

15. Procédé selon la revendication 14, dans lequel l'additif alimentaire est utilisé dans une étape ultérieure pour la fabrication d'une composition d'aliment pour animaux.
